# EUROPEAN PATENT APPLICATION

(11) **EP 3 949 999 A1**
(43) Date of publication of application: **09.02.2022**
(21) Application number: 20784786.4
(22) Date of filing: 03.04.2020
(51) Int. Cl.: A61K 45/00, A61K 8/25, A61K 8/27, A61K 8/29, A61K 8/49, A61K 8/73, A61K 8/96, A61K 31/409, A61K 33/24, A61K 33/30, A61K 41/00, A61K 47/04, A61K 47/36, A61P 17/00, A61P 43/00, A61Q 17/04

(54) **COMPOSITION CONTAINING ULTRAVIOLET WAVELENGTH-CONVERTING SUBSTANCE AND HYDROPHOBIZED SILICA AND/OR HYDROPHOBIZED STARCH**

(30) Priority: 05.04.2019 JP 2019072750
(71) Applicant: Shiseido Company, Ltd., Chuo-ku Tokyo 104-0061 (JP)
(72) Inventor: SUGIHARA, Megumi, Tokyo 104-0061 (JP); ITO, Ryoya, Tokyo 104-0061 (JP); MIYAZAWA, Kazuyuki, Tokyo 104-0061 (JP); KANEMARU, Tetsuya, Tokyo 104-0061 (JP); GILLET, Renaud, Tokyo 104-0061 (JP); MCCARTHY, Bianca, Tokyo 104-0061 (JP)
(74) Representative: Cabinet Beau de Loménie
(86) International application number: PCT/JP2020/015427
(87) International publication number: WO 2020/204195

(57) **Abstract**

There is provided a novel composition containing a UV wavelength conversion substance and a powder of a hydrophobized silica and/or a hydrophobized starch.

## Description

### FIELD

The present invention relates to a composition having a cell activation action and comprising a UV wavelength conversion substance as well as a hydrophobized silica and/or a hydrophobized starch.

### BACKGROUND

Ultraviolet is considered to generate free radicals *in vivo* and thereby cause oxidation of sebum and damage to cellular DNA. Examples of the damage caused by ultraviolet to skin include adverse effects such as skin cancer, photoaging, spots, wrinkles, and inflammation. These are undesirable from a health and beauty perspective. Although ultraviolet has been used for the purpose of sterilization, it is currently focused on protection from rather than active use of ultraviolet in consideration of the balance with harmful effects caused by ultraviolet.

Thus, many measures have been taken to protect skin from ultraviolet. Examples thereof include use of sunscreens, indoor activities avoiding sunlight, and use of UV-cut hats or clothing, and ultraviolet cut films.

For example, PTL 11 describes a fluorescent cosmetic containing a fluorescent calcium aluminate manganese and silica anhydride, which is a silica, in Example 8, but does not describe a UV wavelength conversion substance for bringing about a cell activation effect. The present inventors have found that powders of silica and cornstarch enhance the function of a UV wavelength conversion substance (Japanese Patent Application No. 2019-239802). However, hydrophobized silica and hydrophobized starch are not described.

### [CITATION LIST]

### [PATENT LITERATURE]

[PTL 1] Japanese Registered Patent Publication No. 6424656
[PTL 2] Japanese Registered Patent Publication No. 6361416
[PTL 3] WO 2018/004006
[PTL 4] Japanese Unexamined Patent Publication (Kokai) No. 2018-131422
[PTL 5] Japanese Unexamined Patent Publication (Kokai) No. 5-117127
[PTL 6] Japanese Registered Patent Publication No. 4048420
[PTL 7] Japanese Registered Patent Publication No. 4677250
[PTL 8] Japanese Registered Patent Publication No. 3303942
[PTL 9] Japanese Unexamined Patent Publication (Kokai) No. 2017-88719
[PTL 10] WO 2018/117117
[PTL 11] WO 2017/142057

### SUMMARY

### [TECHNICAL PROBLEM]

An object of the present invention is to provide a novel composition having a cell activation action utilizing ultraviolet.

### [SOLUTION TO PROBLEM]

The present inventors have conducted intensive studies so that ultraviolet can be effectively used for skin. As a result, the present inventors have discovered that the function of a UV conversion substance was significantly enhanced by imparting hydrophobization to a powder of silica or starch, and have conceived of a composition containing these components and a UV wavelength conversion substance.

The present application provides the following inventions.
(1) A composition comprising (A) a UV wavelength conversion substance and (B) a hydrophobized silica and/or a hydrophobized starch.
(2) The composition according to (1), wherein the (A) UV wavelength conversion substance comprises an inorganic UV wavelength conversion substance.
(3) The composition according to (2), wherein the inorganic UV wavelength conversion substance comprises a magnesium titanate phosphor and/or a zinc oxide phosphor.
(4) The composition according to (1), wherein the (A) UV wavelength conversion substance comprises an organic UV wavelength conversion substance.
(5) The composition according to (4), wherein the organic UV wavelength conversion substance comprises one or more selected from the group of phycocyanin, phycoerythrocyanin, phycoerythrin, vitamin A, β-carotene, vitamin K, vitamin B1, vitamin B2, vitamin B2 derivatives, vitamin B6, vitamin B12, folic acid, niacin, salicylic acid, lycopene, gardenia color, capsicum color, capsicum extract, paprika color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a phosphor doped with a sulfur-containing compound, a magnesium titanate phosphor, and a calcium phosphate phosphor.
(6) The composition according to any one of (1) to (5), comprising both an inorganic UV wavelength conversion substance and an organic UV wavelength conversion substance as the (A) UV wavelength conversion substance.
(7) A composition comprising (A') one or more substances selected from the group of phycocyanin, phycoerythrocyanin, phycoerythrin, vitamin A, β-carotene, vitamin K, vitamin B1, vitamin B2, vitamin B2 derivatives, vitamin B6, vitamin B12, folic acid, niacin, salicylic acid, lycopene, gardenia color, capsicum color, capsicum extract, paprika color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a phosphor doped with a sulfur-containing compound, a magnesium titanate phosphor, and a calcium phosphate phosphor, and
(B) a hydrophobized silica and/or a hydrophobized starch.

(8) The composition according to (7), wherein the (A') substance comprises one or more selected from the group of a zinc oxide phosphor, a magnesium titanate phosphor, phycocyanin, and vitamin B2.

(9) The composition according to any one of (1) to (8), wherein the (B) hydrophobized silica has an average particle size of 1 to 10 µm.

(10) The composition according to any one of (1) to (9), wherein the (B) hydrophobized silica is fatty acid soap treated silica, fatty acid ester treated silica, higher alcohol treated silica, silicone treated silica, or fatty acid treated silica.

(11) The composition according to any one of (1) to (10), wherein the (B) hydrophobized starch comprises alkenylsuccinic acid treated starch.

(12) The composition according to any one of (1) to (11), further comprising (C) a UV absorber and/or (D) a UV scattering agent.

(13) The composition according to any one of (1) to (12), which is a sunscreen cosmetic.

(14) The composition according to any one of (1) to (13), which exhibits a fluorescence intensity increasing effect.

(15) The composition according to any one of (1) to (14), which exhibits a cell activation effect.

### [ADVANTAGEOUS EFFECT OF INVENTION]

The UV wavelength conversion substance of the present invention is suitable for effectively utilizing ultraviolet to activate skin cells. The constituent composition of the composition of the present invention is suitable so that the UV wavelength conversion substance converts ultraviolet to visible light. Conventionally, since ultraviolet is not preferable for skin, it is a technical common knowledge in this field to take measures to avoid exposing skin to ultraviolet as much as possible. However, the present invention is based on the knowledge that conversely a UV wavelength conversion substance utilizes ultraviolet to activate cells and thereby provide a preferable action on skin, and is surprising. Thus, the composition of the present invention may lead to an improvement in the quality of life, such that even those who have avoided ultraviolet as much as possible for a reason of beauty or health may feel like going out actively.

### BRIEF DESCRIPTION OF DAWINGS

FIG. 1 is a schematic diagram of Experiments 1 and 2.
FIG. 2 shows the cell activity at UV irradiation using each ultraviolet in Experiment 1. The vertical axis indicates relative fluorescence intensity (au).
FIG. 3 shows the cell activity at UV irradiation of each intensity using C-phycocyanin in each concentration in Experiment 2 as the relative fluorescence intensity (au).
FIG. 4 is a schematic diagram of Experiment 3.
FIG. 5 shows the cell activity when irradiating cells having cell activity temporarily reduced in Experiment 3 with UV using C-phycocyanin as relative fluorescence intensity (au) (P test).

### DESCRIPTION OF EMBODIMENTS

The present invention will be described below in detail with reference to specific embodiments. However, the present invention is not limited to the following embodiments, and can be carried out in any embodiments without departing from the spirit of the present invention.

All of the patent publications, unexamined patent publications, and non-patent literature cited in the present disclosure are incorporated by reference in their entirety into the present disclosure for all purposes.

In the present disclosure, "to" when applied to numerical values refers to a range of values that fall within a range that is greater than or equal to a defined reference value and less than or equal to a defined reference value.

### (A) UV wavelength conversion substance

The composition of the present invention contains a UV wavelength conversion substance as an active component. The phrase "UV wavelength conversion substance" refers to a substance which converts the wavelength of ultraviolet contained in incident light to outgoing light with a wavelength longer than the wavelength of ultraviolet. The phrase "organic UV wavelength conversion substance" refers to a UV wavelength conversion substance which is an organic compound, and the phrase "inorganic UV wavelength conversion substance" refers to a UV wavelength conversion substance which is an inorganic compound.

The ultraviolet may contain UVA, UVB, or UVC. In one embodiment, the ultraviolet is light having a peak wavelength of 200 nm to 400 nm. Further, incident light, for example, sunlight, may contain ultraviolet. Furthermore, the incident light may be ultraviolet or artificially generated ultraviolet may be used.

The outgoing light emitted from the UV wavelength conversion substance has a longer wavelength than ultraviolet and has a peak wavelength of preferably 500 nm to 700 nm. The outgoing light may have one or more peaks, for example, but not limited to, at 510 nm, 520 nm, 530 nm, 540 nm, 550 nm, 560 nm, 570 nm, 580 nm, 590 nm, 600 nm, 610 nm, 620 nm, 630 nm, 640 nm, 650 nm, 660 nm, 670 nm, 680 nm, 690 nm, 700 nm, or within any range therebetween or may be red light, orange light, green light, or blue light. In one embodiment, the main wavelength exhibited by the light emitted from the UV wavelength conversion substance when excited by 200 nm to 400 nm excitation light is 500 nm to 700 nm.

Examples of the UV wavelength conversion substance include the following components: phycobiliproteins such as phycocyanin (allophycocyanin, C-phycocyanin, R-phycocyanin), phycoerythrocyanin, phycoerythrin (B-phycoerythrin, b-phycoerythrin, C-phycoerythrin, R-phycoerythrin); natural or synthetic components such as vitamin A, β-carotene, vitamin K, vitamin B1, vitamin B2, vitamin B6, vitamin B 12, folic acid, niacin, salicylic acid, lycopene, capsicum extract, capsicum color, paprika color, gardenia color, safflower color, turmeric color, cochineal color, perilla color, red cabbage color, flavonoid, carotenoid, quinoid, porphyrins, anthocyanins, and polyphenols; colors such as Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, and meta-aminophenol; phosphors of an inorganic compound doped to exhibit a fluorescence property, such as the blue phosphor containing an amorphous silica particle, cerium, and phosphorus and/or magnesium described in Japanese Registered Patent Publication No. 6424656, the red phosphor containing a europium-activated compound of a mixed crystal of an alkaline earth metal sulfide and a gallium compound described in Japanese Registered Patent Publication No. 6361416; the zinc oxide phosphor described in WO 2018/004006; the zinc oxide phosphor described in Japanese Unexamined Patent Publication (Kokai) No. 2018-131422; and the inorganic phosphor described in Japanese Unexamined Patent Publication (Kokai) No. 5-117127 (hereinafter, a phosphor derived from zinc oxide is referred to as a "zinc oxide phosphor"). In one embodiment, the inorganic phosphor is one or more phosphors selected from phosphors of zinc oxide represented by ZnO:Zn, Zn_{1 + z}, or ZnO_{1 - x}, doped with a sulfide and/or a sulfate, such as zinc sulfide and zinc sulfate, described in WO 2018/004006; magnesium titanate phosphors of a magnesium titanate such as MgTiO₃ or Mg₂ TiO₄ doped with manganese (hereinafter referred to as magnesium titanate is referred to as a "magnesium titanate phosphor"); and calcium phosphate phosphors of a calcium phosphate such as Ca(H₂PO₄)₂, CaHPO₄, or Ca₃ (PO₄)₂ doped with cerium.

Further, the inorganic UV wavelength conversion substance which is an inorganic phosphor may be subjected to a surface treatment. Examples of the surface treatment include a silane compound treatment (octyltriethoxysilane), a silicone compound treatment, a fluorine-modified silicone compound treatment, a fluorine compound treatment, a higher fatty acid treatment (stearic acid, etc.), a higher alcohol treatment, a fatty acid ester treatment, a metal soap treatment, an amino acid treatment, and an alkyl phosphate treatment.

UV wavelength conversion substance may be obtained by a method of extraction from natural products such as animals, plants, and algae or an artificial method such as chemical synthesis. For example, phycobiliproteins may be prepared from blue-green algae such as *Spirulina platensis,* red algae such as *Porphyridium cruentum,* and other algae by the extraction method described in, for example, Japanese Registered Patent Publication No. 4048420, Japanese Registered Patent Publication No. 4677250, or Japanese Registered Patent Publication No. 3303942. Zinc oxide phosphors may be produced by the method described in, for example, WO 2018/004006, Japanese Unexamined Patent Publication (Kokai) No. 2018-131422, or Japanese Unexamined Patent Publication (Kokai) No. 5-117127. Magnesium titanate phosphors may be produced by the method described in Japanese Unexamined Patent Publication (Kokai) No. 2017-88719. Calcium phosphate phosphor may be produced by the method described in WO 2018/117117.

These UV wavelength conversion substances may consist of or contain these exemplified components, which may be used alone or in combination of two or more, as long as the wavelength conversion effect of the invention is not impaired. For example, to the phycobiliprotein or inorganic phosphor, there may be added another wavelength conversion substance such as vitamin B (vitamin B1, vitamin B2, vitamin B6, or vitamin B12) to aim for a synergistic effect. Note that these components are merely examples and any substance which can bring about a wavelength conversion effect can be used in the present invention.

Any derivative of vitamin B2, which is a UV wavelength conversion substance, may be used as long as the derivative is a UV wavelength conversion substance. Examples of the vitamin B2 derivatives include riboflavin acetate ester, riboflavin butyrate, riboflavin phosphate (may be a sodium or mono-diethanolamine salt), flavin mononucleotide, flavin adenine dinucleotide, riboflavin tetrabutyrate, and riboflavin tetranicotinate. Derivatives of lixoflavin, which is a stereoisomer of riboflavin, may be used.

The content of the UV wavelength conversion substance in the composition of the present invention is not particularly limited as long as the wavelength conversion effect of the present invention is not impaired. The content can be appropriately determined in accordance with the type of the UV wavelength conversion substance or the application of the composition containing the UV wavelength conversion substance. The range thereof is not limited and may be, for example, 0.001 to 99.99% by weight, 0.001 to 10% by weight, 0.01 to 99.99% by weight, 0.01 to 10% by weight, 0.1% to 99.9% by weight, or 0.1 to 10% by weight.

As one aspect of the present invention, the UV wavelength conversion substance of the composition contains a zinc oxide phosphor. In the composition of the present invention, preferably, the content of a zinc oxide phosphor relative to the total of the composition is 0.1% by weight or more, preferably 1.0% by mass or more, more preferably 1.5% by weight or more, or even more preferably 2% by weight or more, and 20% by weight or less, preferably 15% by weight or less, more preferably 10% by weight or less, or even more preferably 5% by weight or less. The content relative to the total of the composition is 0.01 to 99.99% by weight, 0.1 to 99.9% by weight, 0.1 to 50% by weight, 0.1 to 40% by weight, 0.1 to 30% by weight, 0.1 to 20% by weight, 0.1 to 10% by weight, or 1 to 10% by weight.

As one aspect of the present invention, the UV wavelength conversion substance of the composition contains a magnesium titanate phosphor. In the composition of the present invention, preferably, the content of a magnesium titanate phosphor relative to the total of the composition is 0.1% by weight or more, preferably 1.0% by mass or more, more preferably 1.5% by weight or more, or even more preferably 2% by weight or more, and 20% by weight or less, preferably 15% by weight or less, more preferably 10% by weight or less, or even more preferably 5% by weight or less. The content relative to the total of the composition is 0.01 to 99.99% by weight, 0.1 to 99.9% by weight, 0.1 to 50% by weight, 0.1 to 40% by weight, 0.1 to 30% by weight, 0.1 to 20% by weight, 0.1 to 10% by weight, 0.5 to 10% by weight, or 1 to 10% by weight.

As one aspect of the present invention, the UV wavelength conversion substance of the composition contains phycocyanin. In the composition of the present invention, preferably, the content of a phycocyanin relative to the total of the composition is 0.00001% by weight or more, preferably 0.0001% by mass or more, and 20% by weight or less, preferably 15% by weight or less, more preferably 10% by weight or less, or even more preferably 5% by weight or less. The content relative to the total of the composition is 0.00001 to 99.99% by weight, 0.0001 to 99.9% by weight, 0.0001 to 50% by weight, 0.0001 to 40% by weight, 0.0001 to 30% by weight, 0.0001 to 20% by weight, 0.0001 to 10% by weight, 0.0001 to 5% by weight, 0.001 to 5% by weight, 0.01 to 5% by weight, 0.05 to 5% by weight, 0.1 to 5% by weight, 0.1 to 3% by weight, or 0.1 to 1% by weight.

As one aspect of the present invention, the UV wavelength conversion substance of the composition is vitamin B2. In the composition of the present invention, preferably, the content of vitamin B2 relative to the total of the composition is 0.00001% by weight or more, preferably 0.0001% by weight or more, and 20% by weight or less, preferably 15% by weight or less, more preferably 10% by weight or less, or even more preferably 5% by weight or less. The content relative to the total of the emulsified composition is 0.00001 to 99.99% by weight, 0.0001 to 99.9% by weight, 0.0001 to 50% by weight, 0.0001 to 40% by weight, 0.0001 to 30% by weight, 0.0001 to 20% by weight, 0.0001 to 10% by weight, 0.0001 to 5% by weight, 0.001 to 5% by weight, 0.01 to 5% by weight, 0.05 to 5% by weight, 0.1 to 5% by weight, 0.1 to 3% by weight, or 0.1 to 1% by weight.

Examples of cell activation include, but are not limited to, promoting metabolism and turnover, improving a function, promoting proliferation, inhibiting oxidation, improving resistance to fatigue and external stimuli, and inhibiting loss of function and activity in cells, such as dermal fibroblasts and/or keratinocytes, of animals including humans. When skin cells are activated, effects such as prevention of or improvement from wrinkles, spots, skin aging, and photoaging can be expected.

The cell activation effect may be measured by measuring, for example, the viability, reducing ability, or proliferation of living cells using AlamarBlue as in the Examples. Any method can be used such as another dye assay, mitochondrial membrane potential-dependent dye assay, intracellular cytochrome c assay, elastase cleavage dye assay, ATP, ADE assay, glycolytic flux and oxygen consumption assay, collagen assay, photoaging assay, collagen glycation assay, inflammatory substances (interleukin 1α, interleukin 8, tumor necrosis factor α) assay, skin barrier function-related protein (corneodesmosin, sphingomyelin phosphodiesterase, filaggrin, involucrin, loricrin, transglutaminase 1, caspase 14) assay, angiogenesis modulator (VEGF-A, ANGPT1) assay, oxidation and/or skin stress-related protein (aromatic hydrocarbon receptor repressor, cytochrome P4501B1, aromatic hydrocarbon receptor repressor, heme oxygenase 1) assay, or hyaluronic acid assay.

The composition of the present invention is suitable for performing the function of a UV wavelength conversion substance and for alleviating, or more positively improving or restoring skin damage during and after ultraviolet irradiation by activating cells. Specifically, the composition of the present invention is suitable for collagen production or hyaluronic acid production by fibroblasts and inhibition of damage caused by photoaging and for inhibiting oxidation stress of keratinocytes, enhancing a barrier function, suppressing an inflammatory reaction, and suppressing the glycation of collagen and angiogenesis in skin.

Fluorescence intensity can be measured using a spectrofluorometer by irradiating with ultraviolet a coating film of the composition on the surface of a substrate, as in the Examples. The substrate may be a resin substrate composed of a polymethyl methacrylate (PMMA), nylon, or acrylic plate or an inorganic plate of glass or quartz. For example, a PMMA plate having V-shaped grooves on its surface (also refer to as "S plate": Japanese Registered Patent Publication No. 4453995) can be used. As the fluorescence intensity, a fluorescence value at a specific single wavelength or an integrated value in a specific wavelength region may be used.

One aspect of the composition of the present invention is a composition containing:
(A') one or more substances selected from the group of phycocyanin, phycoerythrocyanin, phycoerythrin, vitamin A, β-carotene, vitamin K, vitamin B1, vitamin B2, vitamin B2 derivatives, vitamin B6, vitamin B12, folic acid, niacin, salicylic acid, lycopene, gardenia color, capsicum color, capsicum extract, paprika color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a phosphor doped with a sulfur-containing compound, a magnesium titanate phosphor, and a calcium phosphate phosphor, and
(B) a hydrophobized silica and/or a hydrophobized starch.

One aspect of the composition of the present invention is a composition containing (A') one or more substances selected from the group of allophycocyanin, C-phycocyanin, R-phycocyanin, phycoerythrocyanin, B-phycoerythrin, b-phycoerythrin, C-phycoerythrin, R-phycoerythrin, vitamin A, β-carotene, vitamin K, vitamin B1, vitamin B2, vitamin B2 derivatives, vitamin B6, vitamin B12, folic acid, niacin, salicylic acid, lycopene, gardenia color, capsicum color, capsicum extract, paprika color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a phosphor doped with a sulfur-containing compound, a magnesium titanate phosphor, and a calcium phosphate phosphor, and (B) a hydrophobized silica and/or a hydrophobized starch.

The (A') substance to be contained in one aspect of the composition of the present invention contains, for example, a zinc oxide phosphor, a magnesium titanate phosphor, phycocyanin, vitamin B2, or combinations of these. The (A') substance to be contained in one aspect of the composition of the present invention preferably contains a zinc oxide phosphor, a magnesium titanate phosphor, phycocyanin, vitamin B2, or combinations of these. These (A') substances may be subjected to a surface treatment such as a hydrophobization treatment.

### (B) Hydrophobized silica

One aspect of the composition of the present invention contains hydrophobized silica. The term "hydrophobized silica" refers to a silica which has been surface-treated with a hydrophobic inorganic or organic substance (also referred to as "hydrophobized silica" or "hydrophobic silica"), and basically refers to silica fine particles having hydrophobic groups in place of surface hydroxyl groups.

Any known method can be used as the method for hydrophobizing the silica for the hydrophobized silica contained in one aspect of the composition of the present invention. Examples of the method include a silicone treatment, fatty acid treatment, fatty acid soap treatment, fatty acid ester treatment, and higher alcohol treatment, and examples of methods for obtaining hydrophobized silica include a method of hydrophobizing and drying a wet-processed silica sol to obtain hydrophobized silica, a method of adding a silazane compound or a monofunctional silane compound to a hydrophilic silica particle dispersion to triorganosilylate the surface of the silica particles to obtain hydrophobized silica, a method of hydrolyzing and condensing a tetrafunctional silane compound to obtain a hydrophilic silica particle dispersion, followed by replacing the hydrophilic organic solvent with water, then hydrophobizing with a trifunctional silane compound, thereafter replacing the dispersion medium with a ketone solvent, and triorganosilylating the reactive groups remaining on the surface of the silica particles with a silazane compound or a monofunctional silane compound to obtain hydrophobized silica, or a method of adding a trifunctional silane compound to a hydrophilic silica particle dispersion to impart hydrophobicity, and then adding a monofunctional silane compound to obtain hydrophobized silica, though the methods are not limited thereto. These hydrophobization treatments can be carried out according to a conventional method.

Examples of the silicone treatment of silica include treatment with a silicone oil such as methylhydrogenpolysiloxane, dimethylpolysiloxane, methylphenylpolysiloxane, or amino-modified silicone; an alkylalkoxysilane such as methyltrialkoxysilane, ethyltrialkoxysilane, dimethyldialkoxysilane, trimethylalkoxysilane, propyltrialkoxysilane, hexyltrialkoxysilane, or long-chain alkyltrialkoxysilane; or a haloalkylsilane such as trifluoromethylethyltrimethoxysilane, heptadecafluorodecyltrimethoxysilane, perfluoroalkylsilane, methyltrichlorosilane, dimethyldichlorosilane, trimethylchlorosilane, ethyltrichlorosilane, propyltrichlorosilane, hexyltrichlorosilane, and long-chain alkyltrichlorosilane. dimethylpolysiloxane, methylphenylpolysiloxane, methylhydrogenpolysiloxane, amino-modified silicone

Examples of the fatty acid treatment of silica include treatment with palmitic acid, isostearic acid, stearic acid, lauric acid, myristic acid, behenic acid, oleic acid, rosinic acid, or 12-hydroxystearic acid.

Examples of the fatty acid soap treatment of silica include treatments with aluminum stearate, calcium stearate, or 12-hydroxystearate.

Examples of the fatty acid ester treatment of silica include treatments with a dextrin fatty acid ester, a cholesterol fatty acid ester, a sucrose fatty acid ester, or a starch fatty acid ester.

As the hydrophobized silica contained in one aspect of the composition of the present invention, a hydrophobized silica that enhances the function of a UV wavelength conversion substance is preferable, and examples thereof include dimethicone-treated silica, amodimethicone-treated silica, stearic acid- or another higher fatty acid-treated silica, and higher alcohol-treated silica.

The average particle size of the hydrophobized silica used in one aspect of the present invention, from the viewpoint of enhancing the function of the UV wavelength conversion substance, is preferably 10 nm or more, more preferably 100 nm or more, further preferably 1 µm or more, and particularly preferably 1 to 10 µm, 1 to 15 µm, or 1 to 20 µm. When the average particle size of the hydrophobized silica is 1 µm or more, it has a strong effect of enhancing the function of the UV wavelength conversion substance and is suitable for cell activation.

The oil absorption amount of the hydrophobized silica used in one aspect of the present invention is preferably 10 to 400 ml/100 g, and more preferably 80 to 200 ml/100 g. When the oil absorption amount of the hydrophobized silica is 100 to 400 ml/100 g, the feeling of use of cosmetics and quasi-drugs containing the oily composition according to one aspect of the present invention is expected to become non-sticky after application. This is suitable for applications such as cosmetics and quasi-drugs that do not leave a feeling of use. Further, when the oil absorption of the hydrophobized silica is 10 to 100 ml/100 g, the feeling of use of cosmetics and quasi-drugs containing the oily composition, which is one aspect of the present invention, becomes powder-free. This is suitable for applications in cosmetics and quasi-drugs where moisturizing properties are preferred.

The content of the hydrophobized silica used in one aspect of the present invention relative to the total of the composition, from the viewpoint of enhancing the function of the UV wavelength conversion substance, is 0.1% by weight or more, preferably 1% by weight or more, more preferably 2% by weight or more, or further preferably 3% by weight or more, and is 20% by weight or less, preferably 15% by weight or less, more preferably 10% by weight or less, and further preferably 5% by weight or less. The content relative to the total of the composition is 0.01 to 99.99% by weight, 0.1 to 99.9% by weight, 0.1 to 50% by weight, 0.1 to 40% by weight, 0.1 to 30% by weight, 0.1 to 20% by weight %, 0.1 to 10% by weight, 0.5 to 10% by weight, 1 to 10% by weight, 1.5 to 10% by weight, or 0.8 to 5% by weight.

### (B) Hydrophobized starch

One aspect of the composition of the present invention contains hydrophobized starch. The term "hydrophobized starch" refers to a hydrophobic starch treated with a saturated fatty acid or an alkenyl succinic acid.

As the method for saturated fatty acid treatment of starch for the hydrophobized starch contained in one aspect of the composition of the present invention, any known method (e.g., the method described in Japanese Unexamined Patent Publication (Kokai) No. 2018-076277) can be used. As the saturated fatty acid as the surface treatment agent, saturated fatty acids such as capric acid, lauric acid, myristic acid, palmitic acid and stearic acid and saturated fatty acid metal salts such as zinc salt, magnesium salt and aluminum salt of these saturated fatty acids are preferable, and among these, magnesium stearate is preferable because it provides sufficient hydrophobicity.

As a method for treating starch with alkenylsuccinic acid for the hydrophobized starch contained in one aspect of the composition of the present invention, any known method (e.g., the method described in US Patent Publication No. 2613206) can be used. The alkenyl succinic acid that esterifies starch is preferably an alkenyl succinic acid having 5 to 22 carbon atoms, and examples thereof include octenyl succinic acid, decenyl succinic acid, dodecenyl succinic acid, tetradecenyl succinic acid, hexadecenyl succinic acid, and octadecenyl succinic acid. Examples of the metal used for the alkenyl succinic acid metal salt of starch include copper, aluminum, zirconium, iron (divalent and trivalent), calcium, and barium. By containing the alkenyl succinic acid ester metal salt of starch, it is possible to obtain a composition having an excellent effect of making pores and fine wrinkles less noticeable more naturally.

The average particle size of the hydrophobized starch used in one aspect of the present invention is not particularly limited, and is preferably 1 to 100 µm. Further, the particle shape is not particularly limited, but hydrophobized starch having an amorphous shape, an elliptical shape, or a spherical shape can be used.

The hydrophobized starch contained in one aspect of the composition of the present invention is preferably a hydrophobized starch that enhances the function of the UV wavelength conversion substance, and includes, for example, aluminum octenyl succinate-treated starches and magnesium stearate-treated starches.

The content of the hydrophobized starch used in one aspect of the present invention relative to the total of the composition, from the viewpoint of enhancing the function of the UV wavelength conversion substance, is 0.1% by weight or more, preferably 1% by weight or more, more preferably 2% by weight or more, or further preferably 3% by weight or more, and is 20% by weight or less, preferably 15% by weight or less, more preferably 10% by weight or less, and further preferably 5% by weight or less. The content relative to the total of the composition is 0.01 to 99.99% by weight, 0.1 to 99.9% by weight, 0.1 to 50% by weight, 0.1 to 40% by weight, 0.1 to 30% by weight, 0.1 to 20% by weight %, 0.1 to 10% by weight, 0.5 to 10% by weight, 1 to 10% by weight, 1.5 to 10% by weight, or 0.8 to 5% by weight.

### (C) UV absorber

UV absorbers absorb incident ultraviolet and are thus considered to indirectly inhibit the function of a UV wavelength conversion substance. However, surprisingly, the composition of the present invention can contain a UV absorber and the function of a UV wavelength conversion substance can be exhibited.

The composition of the present invention may contain a UV absorber. The phrase "UV absorber" refers to a substance which absorbs ultraviolet and converts it to the energy of heat or infrared. The UV absorber that can be used in the present invention is not particularly limited as long as the function of the UV wavelength conversion substance is not directly impaired. Examples of the UV absorber include salicylic acid-based UV absorbers such as homomenthyl salicylate, ethylhexyl salicylate, homosalate, and triethanolamine salicylate; cinnamic acid-based UV absorbers such as 2-ethylhexyl para-methoxycinnamate, glyceryl diparamethoxycinnamate mono-2-ethylhexanoate, methyl 2,5-diisopropylcinnamate, 2,4,6-tris[4-(2-ethylhexyloxycarbonyl)anilino]-1,3,5-triazine, (hereinafter also referred to as "ethylhexyl triazone"), isopentyl trimethoxycinnamate trisiloxane, an isopropyl para-methoxycinnamate-diisopropylcinnamate ester mixture, and a diethanolamine p-methoxyhydrocinnamate salt; benzoylmethane UV absorbers such as 2-phenyl-benzimidazole-5-sulfuric acid, 4-isopropyldibenzoylmethane, and 4-tert-butyl-4'-methoxydibenzoylmethane; octocrylene, 2-ethylhexyl dimethoxybenzylidene dioxoimidazolidine propionate, 1-(3,4-dimethoxyphenyl)-4,4-dimethyl-1,3-pentanedione, cinoxate, methyl-O-aminobenzoate, 3-(4-methylbenzylidene)camphor, octyltriazone, hexyl diethylaminohydroxybenzoyl benzoate, bis(ethylhexyloxyphenol)methoxyphenyl triazine, and bis(benzotriazolyl) tetramethylbutylphenol methylene. One or more selected therefrom can be contained in the composition.

The total content of the UV absorber that can be contained in the composition of the present invention is 0.5% by weight or more, preferably 1% by weight or more, more preferably 5% by weight or more, even more preferably 7% by weight or more, or most preferably 10% by weight or more, relative to the total of the composition to function as a sunscreen and 40% by weight or less, preferably 30% by weight or less, more preferably 25% by weight or less, even more preferably 20% by weight or less, relative to the total of the composition to avoid excessive absorption of ultraviolet contained in incident light. The total content of the UV absorber that can be contained in the composition of the present invention is 0.5 to 40% by weight, 1 to 40% by weight, 1 to 30% by weight, 5 to 40% by weight, 5 to 30% by weight, 5 to 25% by weight, 7 to 30% by weight, 7 to 25% by weight, 7 to 20% by weight, 10 to 30% by weight, 10 to 25% by weight, or 10 to 20% by weight.

### (D) UV scattering agent

UV scattering agents scatter incident ultraviolet and are thus considered to indirectly inhibit the function of a UV wavelength conversion substance. However, surprisingly, the composition of the present invention can contain a UV absorber and the function of a UV wavelength conversion substance can be exhibited.

The composition of the present invention may contain a UV scattering agent. The term "UV scattering agent" refers to a substance which can reflect/scatter ultraviolet and protect skin from ultraviolet. Examples of the material of the UV scattering agent that can be used in the present invention include titanium dioxide, zinc oxide other than component (A) or (A'), iron oxide, zirconium oxide, and aluminum oxide. The UV scattering agent may be fine particles or a composite thereof. The UV scattering agent preferably contains one or more selected from titanium dioxide and zinc oxide other than component (A) or (A').

The titanium dioxide and zinc oxide used as the UV scattering agent may be titanium dioxide and zinc oxide used commonly in cosmetics. Preferably, those having superior dispersibility, such as those subjected to a surface treatment by a publicly known method, as needed, specifically those subjected to a hydrophobic treatment, can be contained in the composition.

Examples of the surface treatment include a silicone treatment with methylhydrogen polysiloxane or methylpolysiloxane; a fluorine treatment with perfluoroalkyl phosphoric acid ester or perfluoroalcohol; an amino acid treatment with N-acylglutamic acid; an alkylalkoxysilane treatment with octyltriethoxysilane or octyltrimethoxysilane; and further, a lecithin treatment; a metal soap treatment; a fatty acid treatment; and an alkyl phosphate treatment. Thereamong, zinc oxide surface-treated with silicone is preferably used.

The silicone used for surface treatment is not particularly limited. Examples thereof include methylpolysiloxane, methylphenylpolysiloxane, methylhydrogen polysiloxane, methylcyclopolysiloxane, octamethylcyclotetrasiloxane, decamethylcyclopentasiloxane, dodecamethylcyclohexasiloxane, octamethyltrisiloxane, tetradecamethylhexasiloxane, dimethylsiloxane-methyl(polyoxyethylene)siloxane-methyl(polyoxypropylene)siloxane copolymer, dimethylsiloxane-methyl(polyoxyethylene) siloxane copolymer, dimethylsiloxane-methyl(polyoxypropylene) siloxane copolymer, dimethylsiloxane-methylcetyloxysiloxane copolymer, dimethylsiloxane-methylstearoxysiloxane copolymer, and various other silicones. The silicone is preferably methylhydrogen polysiloxane or methylpolysiloxane.

The total content of the UV scattering agent that can be contained in the composition of the present invention is 0.1% by weight or more, preferably 0.5% by weight or more, more preferably 1% by weight or more, even more preferably 5% by weight or more, or most preferably 10% by weight or more, relative to the total of the composition to function as a sunscreen and 40% by weight or less, preferably 30% by weight or less, more preferably 25% by weight or less, or even more preferably 20% by weight or less, relative to the total of the composition to avoid excessive scattering of ultraviolet contained in incident light. The total content of the UV scattering agent that can be contained in the composition of the present invention is 0.1 to 40% by weight, 0.1 to 30% by weight, 0.1 to 25% by weight, 0.1 to 20% by weight, 0.5 to 40% by weight, 0.5 to 30% by weight, 0.5 to 25% by weight, 0.5 to 20% by weight, 1 to 40% by weight, 1 to 30% by weight, 1 to 25% by weight, 1 to 20% by weight, 5 to 40% by weight, 5 to 30% by weight, 5 to 25% by weight, 5 to 20% by weight, 10 to 40% by weight, 10 to 30% by weight, 10 to 25% by weight, or 10 to 20% by weight.

### (E) Oil content

The composition of the present invention may contain an oil content. The phrase "oil content" refers to a hydrophobic substance that phase-separates from water, which is a component of the composition of the present invention. The oil content that can be used in the present invention is not particularly limited and contains one or more of, for example, hydrocarbon oils, ester oils, silicone oils, liquid oils, solid fats, and higher alcohols.

Examples of the hydrocarbon oils include liquid paraffin, tetraisobutane, hydrogenated polydecene, olefin oligomer, isododecane, isohexadecane, squalane, and hydrogenated polyisobutene.

Examples of the ester oils include diisopropyl sebacate, octyl palmitate, cetyl isooctanoate (cetyl 2-ethylhexanoate), triethylhexanoin, neopentyl glycol dicaprate, triisostearin, diisostearyl malate, PPG-3 dipivalate, di-2-ethylhexyl succinate, 2-ethylhexyl 2-ethylhexanoate, polyglyceryl-6 octacaprylate, and glyceryl tri(caprylate/caprate).

Examples of the silicone oils include dimethicone, amino-modified polysiloxane, polyether-modified polysiloxane, alkyl-modified polysiloxane (e.g., decamethylcyclopentaicone, methyl polysiloxane), and fluorine-modified polysiloxane.

Examples of the liquid oils include avocado oil, camellia oil, macadamia nut oil, mink oil, olive oil, castor oil, jojoba oil, triglycerin, and glycerol trioctanoate.

Examples of the solid fats include coconut oil, hardened coconut oil, palm oil, beef tallow, mutton tallow, Japan wax, and hardened castor oil.

Examples of the higher alcohols include isostearyl alcohol, oleyl alcohol, and a butylene glycol-propylene glycol copolymer (e.g., PBG/PPG-9/1 copolymer).

The total content of the oil content that can be contained in the composition of the present invention is 5% by weight or more, preferably 10% by weight or more, more preferably 15% by weight or more, even more preferably 20% by weight or more, or most preferably 25% by weight or more relative to the total of the composition.

### (F) Organic oil-phase thickener

The composition of the present invention may contain an organic oil-phase thickener. The phrase "organic oil-phase thickener" refers to an organic compound capable of increasing the viscosity of an oil phase. The organic oil-phase thickener which can be used in the present invention is not particularly limited as long as the function of the UV wavelength conversion substance is not impaired. The organic oil-phase thickener is preferably a dextrin fatty acid ester, a sucrose fatty acid ester, a glyceryl fatty acid ester, an amino acid-based gelling agent, or a fatty acid or a salt thereof. It is particularly preferable to blend two or more selected therefrom.

The dextrin fatty acid ester is an ester of dextrin or reduced dextrin and a higher fatty acid. Any dextrin fatty acid ester used generally in cosmetics may be used without particular limitation. The dextrin or reduced dextrin preferably has an average degree of sugar polymerization of 3 to 100. A saturated fatty acid having 8 to 22 carbon atoms is preferably used as the constituent fatty acid of the dextrin fatty acid ester. Specific examples thereof include dextrin palmitate, dextrin oleate, dextrin stearate, dextrin myristate, and dextrin palmitate/2-ethylhexanoate.

The fatty acid of the sucrose fatty acid ester is preferably a linear or branched and saturated or unsaturated fatty acid having 12 to 22 carbon atoms. Specific examples thereof include a sucrose caprylic acid ester, sucrose capric acid ester, sucrose lauric acid ester, sucrose myristic acid ester, sucrose palmitic acid ester, sucrose stearic acid ester, sucrose oleic acid ester, sucrose erucic acid ester, and sucrose triacetate tetrastearate.

The glyceryl fatty acid ester is an esterification reaction product obtained by reacting glycerin, a dibasic acid having 18 to 28 carbon atoms, and a fatty acid having 8 to 28 carbon atoms (excluding any dibasic acid). Specific examples thereof include (glyceryl behenate/isostearate/eicosandioate, glyceryl behenate/eicosadioate, and polyglyceryl-10 behenate/eicosadioate.

Examples of the amino acid-based gelling agent include N-lauroyl-L-glutamic acid dibutylamide, dibutyl ethyl hexanoyl glutamide, Polyamide-8, and Polyamide-3.

The fatty acid used may be a solid at ambient temperature. Specific examples thereof include myristic acid, palmitic acid, stearic acid, and behenic acid. Examples of the salt of the fatty acid include calcium salts, magnesium salts, and aluminum salts.

Examples of the vegetable hardened oil include palm kernel hardened oil, hardened castor oil, hydrogenated peanut oil, hydrogenated rapeseed oil, hydrogenated palm oil, hydrogenated camellia oil, hydrogenated soybean oil, hydrogenated olive oil, hydrogenated macadamia nut oil, hydrogenated sunflower oil, hydrogenated wheat germ oil, hydrogenated rice germ oil, hydrogenated rice bran oil, hydrogenated cottonseed oil, and hydrogenated avocado oil.

The preferred organic oil-phase thickener which can contained in the composition of the present invention contains sucrose triacetate tetrastearate, dextrin palmitate, palmitic acid, glyceryl behenate/eicosadioate, N-lauroyl-L-glutamic acid dibutylamide, or Polyamide-8, or a mixture of two or more thereof.

Preferably, the content of the organic oil-phase thickener of the composition of the present invention relative to the total of the composition is 0.01% by weight or more, preferably 0.05% by mass or more, more preferably 0.1% by weight or more, even more preferably 0.3% by weight or more, and is 10% by weight or less, preferably 5% by weight or less, more preferably 3% by weight or less, even more preferably 1% by weight or less. The content relative to the total of the composition is 0.01 to 99.99% by weight, 0.1 to 99.9% by weight, 0.01 to 10% by weight, 0.01 to 5% by weight, 0.05 to 5% by weight, 0.1 to 5% by weight, 0.1 to 3% by weight, 0.1 to 3% by weight, 0.1 to 2% by weight, 0.1 to 1% by weight, or 0.3 to 1% by weight.

### (G) Dispersant

The composition of the present invention may contain a dispersant. The term "dispersant" refers to a substance which adsorbs on the surface of particles (powder) dispersed in a water phase or oil phase whereby the particles can be dispersed homogenously in a water-based or oil-based medium. Preferable examples of the dispersant contained in the composition of the present invention include PEG-10 dimethicone, bis-butyldimethicone polyglyceryl-3, PEG-polydimethylpolysiloxane ethyldimethicone, lauryl PEG-polydimethylpolysiloxane ethyldimethicone, cetyl PEG/PPG-10/dimethicone, isostearic acid, diisostearic acid polyglyceryl-2, carboxydecyl trisiloxane, PEG-12 dimethicone, and polyoxyethylene sorbitan monostearate, and combinations of two or more thereof.

Preferably, the content of the dispersant of the composition of the present invention relative to the total of the composition is 0.1% by weight or more, preferably 1.0% by mass or more, more preferably 1.5% by weight or more, even more preferably 2% by weight or more, and 20% by weight or less, preferably 15% by weight or less, more preferably 10% by weight or less, or even more preferably 5% by weight or less. The content relative to the total of the composition is 0.01 to 99.99% by weight, 0.1 to 99.9% by weight, 0.1 to 50% by weight, 0.1 to 40% by weight, 0.1 to 30% by weight, 0.1 to 20% by weight, 0.1 to 10% by weight, 0.5 to 10% by weight, 1 to 10% by weight, 1.5 to 10% by weight, or 0.8 to 3% by weight.

### (Other optional components)

Various components can be appropriately blended in the composition of the present invention as long as the effect of the present invention is not impaired. Examples of the various components include additive components that can be blended generally in cosmetics, such as clay minerals (dimethyldistearylammonium hectorite), powders (polymethyl methacrylate, crosslinked silicone/network-type silicone block copolymer, silica, hydrophobized talc, cornstarch, hydrophobized polyurethane), chelating agents (disodium edetate hydrate), fragrances, moisturizing agents (glycerin, dipropylene glycol), preservatives, anionic surfactants, cationic surfactants, amphoteric surfactants, nonionic surfactants, water-soluble polymers, silicone-modified polysaccharides, other film-forming agents, metal ion sequestering agents, lower alcohols (ethyl alcohol), polyhydric alcohols, various extracts, sugars, amino acids, organic amines, polymer emulsions, pH adjusters, skin nutrients, vitamins other than (A) or (A'), pharmaceuticals, quasi-drugs, water-soluble drugs applicable to cosmetics, antioxidants, buffering agents, injection agents, pigments, dyes, colors, water, acid components, and alkaline components. These optional components can be appropriately blended in an oil phase or a water phase. Further, another cell activating agent may be contained or co-used to enhance the effect of the present invention.

One aspect of the composition of the present invention is a water-in-oil type composition containing a powder. The water-in-oil type composition of the present invention can be produced by a conventional method.

Specifically, the composition of the present embodiment is obtained in accordance with the following procedures: component (A) or (A'), a powder of (B) and optionally other oil-based components are mixed to prepare an oil phase. Subsequently, water and optionally other water-soluble components are mixed to prepare a water phase. The water phase is added to the oil phase and stirred to obtain a composition.

The composition of the present invention may be an oil-in-water type emulsified composition. The oil-in-water type emulsified composition of the present invention can be produced by a conventional method.

Specifically, the composition of the present embodiment is obtained in accordance with the following procedures: At a temperature of about 25 °C, water-soluble components and water are mixed to prepare a water phase. Separately, at a temperature of about 80 °C, oil-based components such as oil content are mixed to prepare an oil phase. Subsequently, the component (A) or (A') is added to the oil phase and mixed, and thereafter cooled to about 25 °C while stirring. To the water phase were added the oil phase and other components, followed by stirring to obtain a composition.

One aspect of the composition of the present invention is an oil-in-water type composition containing a powder. Examples of the powder include polymethyl methacrylate, crosslinked silicone/network-type silicone block copolymer, hydrophobized talc, and hydrophobized polyurethane. The oil-in-water type emulsified composition containing a powder of the present invention can be produced by a conventional method.

The composition of the present invention can be a cosmetic base or a sunscreen cosmetic such as a sunscreen cream. The formulation form may be, for example, an emulsion, a cream, or a solid stick.

The composition of the present invention can be used by applying it to any of skin, in particular, skin excluding hair, preferably face, body, limbs, preferably by coating any thereof with the composition. For example, by the application, preferably coating, of the composition of the present embodiment, not only can skin be protected from ultraviolet to suppress adverse effects on the skin, the skin can also be imparted with natural and preferable brightness via activation of skin cells.

### EXAMPLES

The present invention will be described in more detail with reference to the following Examples. Note that the present invention is not limited thereto.

Example 1: Cell activation effects of various UV wavelength conversion substances
Experiment 1-1: Preparation of UV wavelength conversion substance
A UV wavelength conversion substance was prepared as follows:

### (1) B-phycoerythrin

B-phycoerythrin was obtained from *Porphiridium Cruentum* extract. The absorption spectrum thereof had a peak wavelength of 305 nm, and the emission spectrum thereof had peak wavelengths of 570 nm and 610 nm.

### (2) C-phycocyanin

C-phycocyanin was obtained from *Spirulina platensis* extract. The absorption spectrum thereof had a peak wavelength of 350 nm, and the emission spectrum thereof had peak wavelengths of 640 nm and 700 nm. Linablue by DIC Corp. was used.

### (3) Zinc oxide phosphor

Lumate G by Sakai Chemical Industry Co., Ltd. was used. Lumate G is a zinc oxide phosphor of ZnO doped with a sulfur-containing compound, as described in WO 2018/004006. The absorption spectrum thereof had a peak wavelength of 365 nm, and the emission spectrum thereof had a peak wavelength of 510 nm.

### (4) Magnesium titanate phosphor

Lumate R by Sakai Chemical Industry Co., Ltd. was used. Lumate R is a magnesium titanate phosphor of MgTiO₃ doped with manganese. The absorption spectrum thereof had a peak wavelength of 365 nm, and the emission spectrum thereof had a peak wavelength within a zone of 660 to 680 nm.

The UV wavelength conversion substances (1) and (2) were dissolved in water to prepare solutions having a concentration of 1% or 5%.

The UV wavelength conversion substances (3) and (4) were dispersed in alcohol to prepare 5% and 10% dispersions.

### Experiment 1-2: Preparation of cell sample

A cell sample was prepared as follows:
1. Human dermal fibroblasts and human skin keratinocytes purchased from Kurabo Industries Ltd. were used. A cell suspension (1 mL) stored in liquid nitrogen was thawed in a water bath (37 °C) to an extent that small ice pellets remained, and then diluted with 9 mL of warm medium.
2. The diluted suspension was mixed gently and then transferred to a T75 flask and incubated overnight at 37 °C.
3. The next day, the medium was replaced with 10 mL of fresh medium.
4. The medium was replaced periodically (once every 2 days for fibroblasts and once every 2 to 3 days for keratinocytes) and cell proliferation was continued. Meanwhile, the cells were observed using a microscope, and it was confirmed that the cells grew in the correct form.
5. After the cells reached about 80% confluence, the cells were passaged. The cells were passaged by washing the cells once with 10 mL of warm PBS, adding 5 mL of warm trypsin to a T75 flask, covering the bottom of the flask with a trypsin solution, followed by aspirating at room temperature for 1 minute.
6. The flask was allowed to stand in an oven at 37 °C for (maximum) 2 minutes for fibroblasts and (maximum) 7 minutes for keratinocytes. The cells were observed using a microscope and confirmed to be small and oval.
7. Thereafter, the side of the T75 flask was lightly tapped to release the cells. The cells were observed using a microscope and confirmed to be moving freely.
8. Fibroblasts were resuspended in 5 mL warm FGM (containing 10% serum) and transferred to a sterile 50 mL Falcon tube. An additional 5 mL of warm FGM was used to flush the flask and added to the Falcon tube to ensure transfer of all cells.
9. The cells were centrifuged at 10,000 rpm for 5 minutes (4 °C) and the supernatant was removed taking care not to disturb the pellet of cells.
10. Depending on the cell type, fibroblasts were resuspended in FGM or KGM at a concentration of 2 × 10⁴ cells/well (500 µ L), and keratinocytes were resuspended in FGM or KGM at a concentration of 4 × 10⁴ cells/well (500 µ L) and plated 24-well plates.
11. Cells were seeded in 24-well plates and the media were changed periodically (once every 2 days for fibroblasts and once every 2 to 3 days for keratinocytes) and the cells were grown until 60 to 70% confluence (depending on the type of experiment) was reached. (Note: Fibroblasts should reach the desired confluency in 24 hours at a cell density of 2 × 10⁴ cells/well. When the cell density is low, for example, 1 × 10⁴ cells/well, it takes 48 hours for fibroblasts to reach the desired confluency.)
12. 24 hours before irradiation, the medium was changed to a supplement-free medium (in the case of keratinocytes) or a medium containing a low concentration of serum (0.5% FCS) (in the case of fibroblasts).

### Experiment 1-3: Ultraviolet irradiation

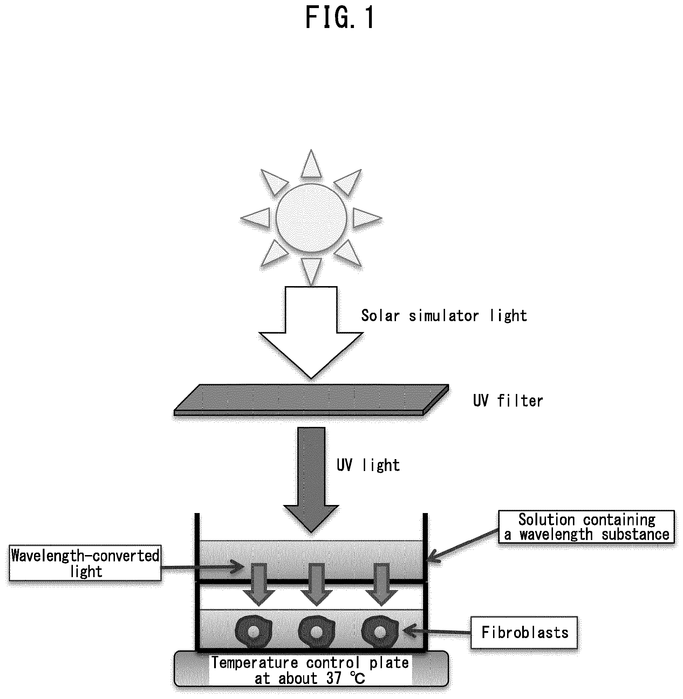

1. A solar simulator was turned on at least 30 minutes before irradiation to warm up a lamp. The solar simulator was set to use a UG11 filter. UG11 filters are filters that allow only UVB to pass and cut light of other wavelengths. The UV light passed through the UG11 filter had a peak wavelength of 300 nm to 385 nm.
2. A temperature control plate was turned on and set to 33 °C.
3. The cells prepared in Experiment 1-2 were washed once with warm PBS.
4. To each well was added 0.5 mL of a warmed Martinez solution (145 mM NaCl, 5.5 mM KCl, 1.2 mM MgCl_{2·}6H₂O, 1.2 mM NaH₂PO₄·2H₂O, 7.5 mM HEPES, 1 mM CaCl₂, and 10 mM D-glucose).
5. As depicted in FIG. 1, cell wells were placed on a plate. Then, 0.4 ml of a solution containing the UV wavelength conversion substances (1) to (4) prepared in Experiment 1-1 was injected into each well of a 24-well plate. The wells containing cells were placed to be covered, such that the cell solution was irradiated with UV light through the solution of the UV wavelength conversion substances without direct contact between the UV wavelength conversion substance solution and the cell solution.
6. Irradiation was carried out to a total dose of 100 mJ/cm². Further, as controls, there were prepared a sample of cells irradiated directly with UV light without placing a plate of a UV wavelength conversion substance on cell wells and a sample of cells cultured in the dark without irradiation with UV light.
7. After irradiation, the Martinez was replaced with warm KGM (without supplements) or FGM (containing 0.5% FCS) and the plate was returned to the incubator at 37 °C.

### Experiment 1-4: Measurement of cell activity

After the completion of Experiment 1-3, the cells maintained in the incubator for 48 hours were used to measure the activity by the following method:
1. A medium (KGM medium without supplements or 0.5% FCS-containing FGM medium) was supplemented with 10% AlamarBlue and warmed to 37 °C. (Keep the solution in the dark.)
2. The medium in the wells was replaced with 500 µL of the above 10% AlamarBlue solution, and the plate was returned to the incubator at 37 °C for about 3 hours. Control wells were also maintained in the incubator. These solutions were maintained in the dark to protect them from light.
3. After 3 hours, 100 µL aliquots were collected and transferred to a black 96-well plate.
4. Fluorescence measurement values at 544 nm/590 nm were read using a fluorometer (OPwave +, Ocean Photonics).

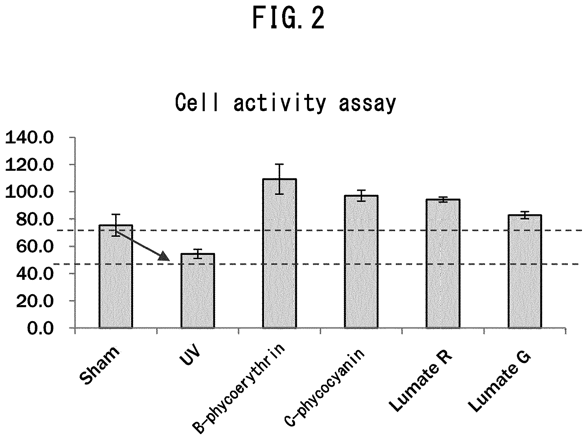

The results are shown in FIG. 2. UV irradiation reduced cell activity compared to the control without irradiation. However, the activity of cells irradiated with UV through any UV wavelength conversion substance was increased compared to the control without irradiation. From the above results, it was found that although the cell activity was decreased by UV irradiation, the decrease in cell activity is suppressed using a UV wavelength conversion substance.

### Example 2: Influence of concentration of UV wavelength conversion substance or intensity of UV on cell activity

The same method as in Experiment 1 was employed except that C-phycocyanin was used as the UV wavelength conversion substance, a cell culture was covered with a plate of a solution containing C-phycocyanin at 0%, 0.4%, or 2%, and irradiated with UV at a dose of 0, 10, 25, 50, 75, or 100 mJ/cm².

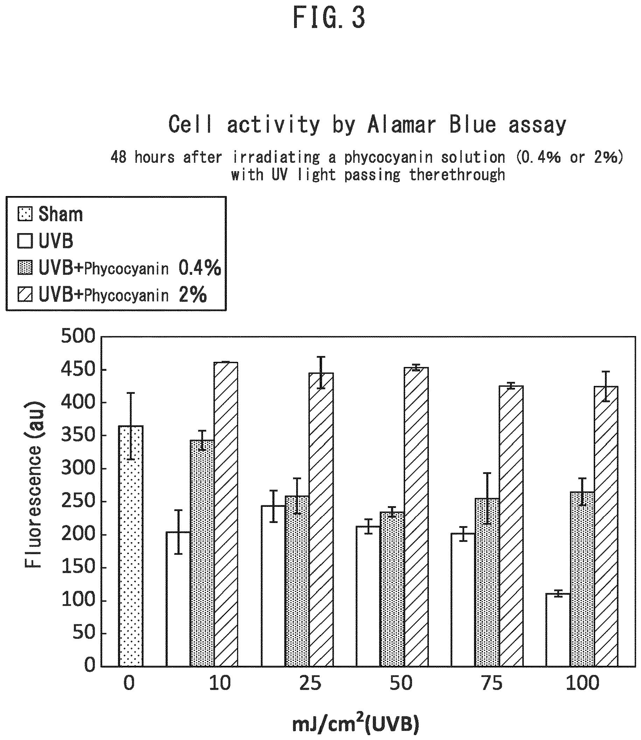

The results are shown in FIG. 3. When the UV wavelength conversion substance was not used, the cell activity decreased as the UV irradiation amount increased. However, when 0.4% C-phycocyanin was added, the decrease in cell activity was suppressed even when UV irradiation was carried out, and when 2% C-phycocyanin was added, the cell activity was even increased compared to the case where UV irradiation was not carried out. From the above results, it was found that although the cell activity was decreased by UV irradiation, the use of a UV wavelength conversion substance not only suppressed the decrease in cell activity in a concentration-dependent manner, but also enhanced the cell activity.

### Example 3: Restoration of cell activity decreased by UV irradiation

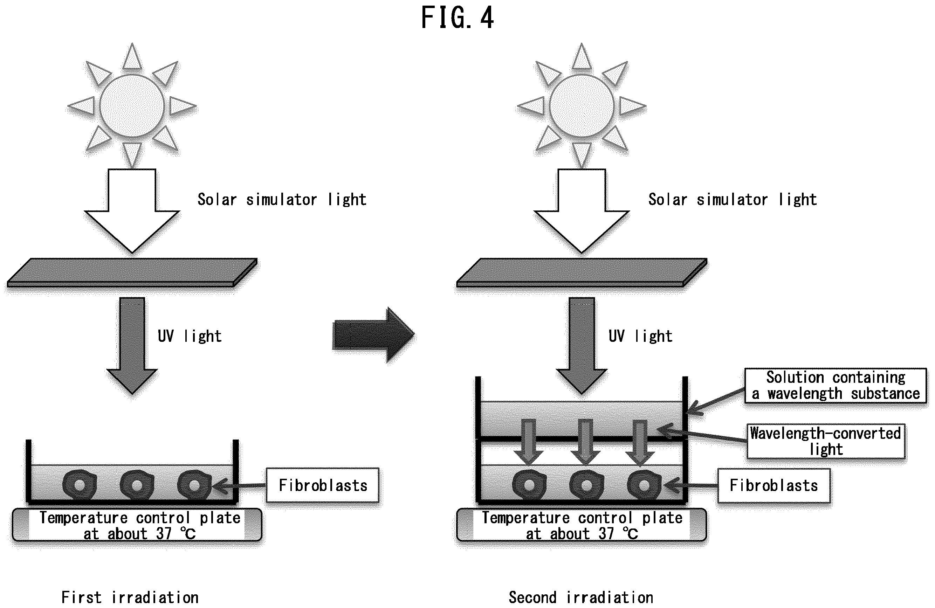

The same method as in Experiment 1 was employed except that as shown in FIG. 4, UV irradiation was carried out without using a UV wavelength conversion substance until the irradiation amount reached 400 mJ/cm² to once decrease the cell activity, and a cell culture was covered with a plate of a solution containing C-phycocyanin as the UV wavelength conversion substance at 0%, 0.4%, or 2%, and irradiated with UV at a dose of 0, 10, 25, 50, 75, 100, or 200 mJ/cm².

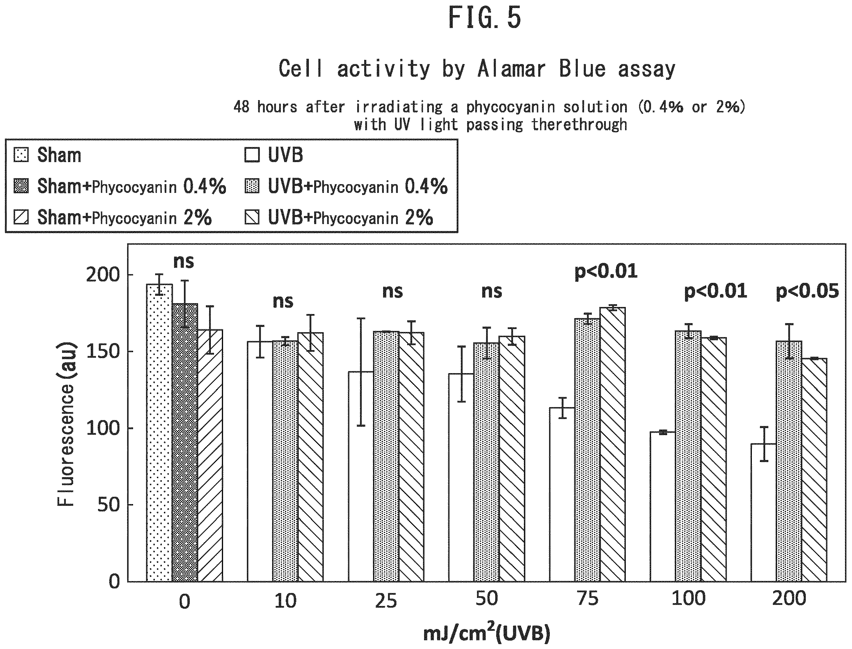

The results are shown in FIG. 5. It can be seen that cell activity was recovered by subjecting even cells having once decreased activity by UV irradiation without using a UV wavelength conversion substance to UV irradiation using a UV wavelength conversion substance. This effect of C-phycocyanin was equivalent at concentrations of 0.4% and 2%, suggesting a sufficient cell activation effect at 0.4%. On the other hand, when UV irradiation was carried out without using a UV wavelength conversion substance, the cell activity decreased in a UV dose-dependent manner.

The results for human dermal fibroblasts are described above. Similar results were also observed for keratinocytes (data not shown). From these results, it was found that a UV wavelength conversion substance not only suppressed a decrease in cell activity due to UV irradiation, but also has an effect of activating cells using UV light. When skin cells are activated, prevention of and improvement from wrinkles, spots, skin aging, photoaging, etc. are expected.

From Examples 1 to 3 above, it was considered that a UV wavelength conversion substance converts the wavelength of ultraviolet, and emitted visible light (fluorescence having a main wavelength of 500 nm to 700 nm) activates skin cells such as fibroblasts and corneocytes. Thus, various compositions containing a UV wavelength conversion substance were produced, and the amount of fluorescence emitted during ultraviolet irradiation was evaluated.

For fluorescence measurement, the composition was applied to an S plate (refer to Japanese Registered Patent Publication No. 4453995) at 2 mg/cm² and dried to prepare a coating film of the composition. The obtained coating film was irradiated with ultraviolet having a predetermined wavelength, and a fluorescence integrated value in a predetermined wavelength region was measured using a fluorescence spectrophotometer RF-5300PC (Shimadzu Corporation). When the UV wavelength conversion substance was a zinc oxide phosphor, a coating film was irradiated with ultraviolet at 365 nm, and the fluorescence integrated value of 400 to 600 nm was measured in the same manner. When the UV wavelength conversion substance was a magnesium titanate phosphor, a coating film was irradiated with ultraviolet at 340 nm, and the fluorescence integrated value of 550 to 800 nm was measured in the same manner. When the UV wavelength conversion substance was C-phycocyanin, a coating film was irradiated with ultraviolet light at 350 nm, and fluorescence integrated values at 550 to 800 nm were measured in the same manner. When the UV wavelength conversion substance was vitamin B2, a coating film was irradiated with ultraviolet at 270 nm, and the fluorescence integrated value at 400 to 750 nm was measured in the same manner.

### Example 4: Effect of hydrophobization of silica

Compositions (Formulation Examples S0 to S4) having the compositions shown in Table 1 were produced according to a conventional production process. Formulation Example S0 contained silica, and Formulation Examples S1 to S4 contained various hydrophobic silicas. Each Formulation Example contained a zinc oxide phosphor, which is a UV wavelength conversion substance. When the fluorescence integrated value of Comparative Example (S0) is 100%, the fluorescence integrated values of Formulation Examples S1 to S4 were 183%, 172%, 152%, and 144%, respectively, and it was found that by hydrophobizing the silica, the wavelength conversion function of the UV wavelength conversion substance was enhanced, depending on the hydrophobization method, and in particular, hydrophobized silica having a large particle size was excellent in the UV wavelength conversion function.

**[Table 1]**

| | Formulation composition | Formulation Example S0 | Formulation Example S 1 | Formulation Example S2 | Formulation Example S3 | Formulation Example S4 |
|---|---|---|---|---|---|---|
| Water | water | 22.75 | 22.75 | 22.75 | 22.75 | 22.75 |
| Alcohol | ethanol | 6 | 6 | 6 | 6 | 6 |
| Moisturizing agent | glycerin | 2.5 | 2.5 | 2.5 | 2.5 | 2.5 |
| | dipropylene glycol | 3 | 3 | 3 | 3 | 3 |
| Clay minerals | dimethyldistearylammoniu m hectorite | 0.15 | 0.15 | 0.15 | 0.15 | 0.15 |
| Surfactant | lauryl PEG-9 pol ydimethylpol ysiloxyeth vl dimethicone | 0.9 | 0.9 | 0.9 | 0.9 | 0.9 |
| | PEG-10 dimethicone | 3 | 3 | 3 | 3 | 3 |
| Oil content | dimethicone | 13.5 | 13.5 | 13.5 | 13.5 | 13.5 |
| | isododecane | 10 | 10 | 10 | 10 | 10 |
| | PBG/PPG-9/1copolymer | 2 | 2 | 2 | 2 | 2 |
| | octyl palmitate | 3 | 3 | 3 | 3 | 3 |
| | methylpolysiloxane | | | | | 2 |
| UV absorber | octocrylene | 3 | 3 | 3 | 3 | 3 |
| | ethylhexyl salicylate | 5 | 5 | 5 | 5 | 5 |
| | homosalate | 5 | 5 | 5 | 5 | 5 |
| UV scattering agent | fine particulate zinc oxide | 10 | 10 | 10 | 10 | 10 |
| Phosphor | zinc oxide phosphor | 5 | 5 | 5 | 5 | 5 |
| Powder | silica | 5 | | | | |
| | dimethicone-treated silica (particle size about 5 µm) | | 5 | | | |
| | amodimethicone-treated silica (particle size about 5 um) | | | 5 | | |
| | stearic acid-treated silica (particle size about 5 µm) | | | | 5 | |
| | dimethylsilylated silica (particle size about 16 nm) | | | | | 3 |
| Chelating agent | chelating agent | 0.2 | 0.2 | 0.2 | 0.2 | 0.2 |
| | fluorescence integrated value | 100% | 183% | 172% | 152% | 144% |

### Example 5: Effect of hydrophobic treatment of starch

Compositions (Formulation Examples DO and D1) having the compositions shown in Table 2 were produced according to a conventional production process. Formulation Example DO contained a starch, and Formulation Example D1 contained a hydrophobized starch (octenyl succinate starch Al). Both Formulation Examples contained a zinc oxide phosphor, which is UV wavelength conversion substance. When the fluorescence integrated value of the Comparative Example (D0) is 100%, the fluorescence integrated value of Formulation Example D1 was 242%, and it was found that the UV wavelength conversion function of the UV wavelength conversion substance was enhanced by hydrophobizing the starch.

**[Table 2]**

| | Formulation composition | Formulation Example DO | Formulation Example D 1 |
|---|---|---|---|
| Water | water | 22.75 | 22.75 |
| Alcohol | ethanol | 6 | 6 |
| Moisturizing agent | glycerin | 2.5 | 2.5 |
| | dipropylene glycol | 3 | 3 |
| Clay minerals | dimethyldistearylammonium hectorite | 0.15 | 0.15 |
| Surfactant | lauryl PEG-9 polydimethylpolysiloxyethyl dimethicone | 0.9 | 0.9 |
| | PEG-10 dimethicone | 3 | 3 |
| Oil content | dimethicone | 13.5 | 13.5 |
| | isododecane | 10 | 10 |
| | PBG/PPG-9/1copolymer | 2 | 2 |
| | octyl palmitate | 3 | 3 |
| UV absorber | octocrylene | 3 | 3 |
| | ethylhexyl salicylate | 5 | 5 |
| | homosalate | 5 | 5 |
| UV scattering agent | fine particulate zinc oxide | 10 | 10 |
| Phosphor | zinc oxide phosphor | 5 | 5 |
| Powder | starch | 5 | |
| | aluminum starch octenylsuccinate (particle size about 15 µm) | | 5 |
| Chelating agent | chelating agent | 0.2 | 0.2 |
| | fluorescence integrated value | 100% | 242% |

### Example 6: Effect of vitamin B2 as UV wavelength conversion substance

Compositions (Formulation Examples VZ0 to VZ2 and VDO and VD1) having the compositions in Table 3 were produced according to a conventional production process. Formulation Example VZ0 contained silica, and Formulation Examples VZ1 and VZ2 contained various hydrophobized silicas. Formulation Example VDO contained a starch, and Formulation Example VD1 contained a hydrophobized starch (octenyl succinate starch Al). Each Formulation Example contained vitamin B2 (riboflavin), which is a UV wavelength conversion substance. When the fluorescence integrated value of the Comparative Example (VZ0) is 100%, the fluorescence integrated values of Formulation Examples VZ1 and VZ2 are 223% and 321%, respectively, and it was found that the hydrophobizing treatment of silica enhances the UV wavelength conversion function of the UV wavelength conversion substance, depending on the hydrophobizing treatment method. When the fluorescence integrated value of the Comparative Example (VD0) is 100%, the fluorescence integrated value of Formulation Example VD1 was 269%, and it was found that the UV wavelength conversion function of vitamin B2 was enhanced by hydrophobizing the starch.

**[Table 3]**

| | Formulation composition | Formulation Example VZ0 | Formulation Example VZ1 | Formulation Example VZ2 | Formulation Example VDO | Formulation Example VD1 |
|---|---|---|---|---|---|---|
| Water | water | 27.74 | 27.74 | 27.74 | 27.74 | 27.74 |
| Alcohol | ethanol | 6 | 6 | 6 | 6 | 6 |
| Moisturizing agent | glycerin | 2.5 | 2.5 | 2.5 | 2.5 | 2.5 |
| | dipropylene glycol | 3 | 3 | 3 | 3 | 3 |
| Clay minerals | dimethyldistearylammonium hectorite | 0.15 | 0.15 | 0.15 | 0.15 | 0.15 |
| Surfactant | lauryl PEG-9 polydimethylpolysiloxyethyl dimethicone | 0.9 | 0.9 | 0.9 | 0.9 | 0.9 |
| | PEG-10 dimethicone | 3 | 3 | 3 | 3 | 3 |
| Oil content | dimethicone | 13.5 | 13.5 | 13.5 | 13.5 | 13.5 |
| | isododecane | 10 | 10 | 10 | 10 | 10 |
| | PBG/PPG-9/lcopolymer | 2 | 2 | 2 | 2 | 2 |
| | octyl palmitate | 3 | 3 | 3 | 3 | 3 |
| UV absorber | octocrylene | 3 | 3 | 3 | 3 | 3 |
| | ethylhexyl salicylate | 5 | 5 | 5 | 5 | 5 |
| | homos alate | 5 | 5 | 5 | 5 | 5 |
| UV scattering agent | fine particulate zinc oxide | 10 | 10 | 10 | 10 | 10 |
| Phosphor | vitamin B2 | 0.01 | 0.01 | 0.01 | 0.01 | 0.01 |
| Powder | silica | 5 | | | | |
| | dimethicone-treated silica (particle size about 5 µm) | | 5 | | | |
| | amodimethicone-treated silica (particle size about 5µm) | | | 5 | | |
| | starch | | | | 5 | |
| | aluminum starch octenylsuccinate (particle size about 15 µm) | | | | | 5 |
| Chelating agent | chelating agent | 0.2 | 0.2 | 0.2 | 0.2 | 0.2 |
| | fluorescence integrated value | 100% | 223% | 321% | 100% | 269% |

### Example 7: Effect of magnesium titanate phosphor as UV wavelength conversion substance

Compositions (Formulation Examples M0 and M1) having the compositions shown in Table 4 were produced according to a conventional production process. Formulation Example M0 contained silica, and Formulation Example M1 contained a hydrophobized silica (dimethicone-treated silica). Each Formulation Example contained a magnesium titanate phosphor (octyltriethoxysilane treated magnesium titanate phosphor), which is a UV wavelength conversion substance. When the fluorescence integrated value of the Comparative Example (M0) is 100%, the fluorescence integrated value of Formulation Example M1 was 181%, and it was found that the wavelength conversion function of the magnesium titanate phosphor was enhanced by hydrophobizing the silica.

**[Table 4]**

| | Formulation composition | Formulation Example M0 | Formulation Example M1 |
|---|---|---|---|
| Water | water | 22.75 | 22.75 |
| Alcohol | ethanol | 6 | 6 |
| Moisturizing agent | glycerin | 2.5 | 2.5 |
| | dipropylene glycol | 3 | 3 |
| Clay minerals | dimethyldistearylammonium hectorite | 0.15 | 0.15 |
| Surfactant | lauryl PEG-9 polydimethylpolysiloxyethyl dimethicone | 0.9 | 0.9 |
| | PEG-10 dimethicone | 3 | 3 |
| Oil content | dimethicone | 13.5 | 13.5 |
| | isododecane | 10 | 10 |
| | PBG/PPG-9/1copolymer | 2 | 2 |
| | octyl palmitate | 3 | 3 |
| UV absorber | octocrvlene | 3 | 3 |
| | ethylhexyl salicylate | 5 | 5 |
| | homosalate | 5 | 5 |
| UV scattering agent | fine particulate zinc oxide | 10 | 10 |
| Phosphor | magnesium titanate phosphor | 5 | 5 |
| Powder | silica | 5 | |
| | dimethicone-treated silica (particle size about 5µm) | | 5 |
| Chelating agent | chelating agent | 0.2 | 0.2 |
| | fluorescence integrated value | 100% | 181% |

### Example 8: Effect of phycocyanin as UV wavelength conversion substance

Compositions (Formulation Examples P0 and P1) having the compositions shown in Table 5 were produced according to a conventional production process. Formulation Example P0 contained silica, and Formulation Example P1 contained a hydrophobized silica (dimethicone-treated silica). Both formulations contained C-phycocyanin, which is A UV wavelength conversion substance. When the fluorescence integrated value of the Comparative Example (P0) is 100%, the fluorescence integrated value of Formulation Example P1 was 169%, and it was found that the UV wavelength conversion function of phycocyanin was enhanced by hydrophobizing the silica.

**[Table 5]**

| | Formulation composition | Formulation Example P0 | Formulation Example P1 |
|---|---|---|---|
| Water | water | 22.75 | 22.75 |
| Alcohol | ethanol | 6 | 6 |
| Moisturizing agent | glycerin | 2.5 | 2.5 |
| | dipropylene glycol | 3 | 3 |
| Clay minerals | dimethyldistearylammonium hectorite | 0.15 | 0.15 |
| Surfactant | lauryl PEG-9 polydimethylpolysiloxyethyl dimethicone | 0.9 | 0.9 |
| | PEG-10 dimethicone | 3 | 3 |
| Oil content | dimethicone | 13.5 | 13.5 |
| | isododecane | 10 | 10 |
| | PBG/PPG-9/1copolymer | 2 | 2 |
| | octyl palmitate | 3 | 3 |
| | methylpolysiloxane | 4 | 4 |
| UV absorber | octocrylene | 3 | 3 |
| | ethylhexyl salicylate | 5 | 5 |
| | homosalate | 5 | 5 |
| UV scattering agent | fine particulate zinc oxide | 10 | 10 |
| Phosphor | phycocyanin | 1 | 1 |
| Powder | silica | 5 | |
| | dimethicone-treated silica (particle size about 5 µm) | | 5 |
| Chelating agent | chelating agent | 0.2 | 0.2 |
| | fluorescence integrated value | 100% | 169% |

As an example of the composition of the present invention, the following compositions shown in Table 6 can be formulated.

**[Table 6]**

| Formulation composition | Content (mass%) |
|---|---|
| Purified water | Total amount of 100 with water |
| Ethanol | 10 |
| EDTA·3Na | 0.1 |
| Sodium chloride | 0.1 |
| Sodium pyrosulfite | 0.01 |
| Glycerin | 2 |
| Xylitol | 1 |
| Tormentilla extract | 1 |
| Sodium hyaluronate | 0.1 |
| 2-O-ethyl-L-ascorbic acid | 0.1 |
| Dipotassium glycyrrhizinate | 0.05 |
| Isododecane | 5 |
| Isopropyl myristate | 3 |
| Diisopropyl sebacate | 10 |
| PBG/PPG-9/1copolymer | 1 |
| Dimethicone (1.5cs) | 8 |
| Dimethicone (6cs) | 2 |
| Cyclopentasiloxane solution with 50% trisiloxysilicic acid | 2 |
| Octocrylene | 5 |
| Hexyl diethylaminohydroxybenzoyl benzoate | 1 |
| Bis(ethylhexyloxyphenol)methoxyphenyl triazine | 0.8 |
| Ethylhexyl salicylate | 5 |
| Homosalate | 3 |
| Fine particulate titanium oxide treated with stearic acid/Al hydroxide(particle sizel5nm) | 3 |
| Distearyldimethylammonium chloride 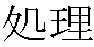 fine particulate zinc oxide | 7 |
| Zinc oxide phosphor | 5 |
| Dimethicone-treated silica | 5 |
| Carnauba waxpowder | 3 |
| PEG-9 polydimethylpolysiloxyethyl dimethicone | 1 |
| Cetyl PEG/PPG-10/1dimethicone | 1 |
| Dimethyldistearylammonium hectorite | 0.2 |
| Isostearic acid | 0.1 |
| Tocopherol | 0.01 |
| Fragrance | q.s. |

The embodiments of the composition of the present invention are described above. However, the present invention is not limited thereto, and can be modified as appropriate without departing from the spirit of the invention.

## Claims

1. A composition comprising (A) a UV wavelength conversion substance and (B) a powder of a hydrophobized silica and/or a hydrophobized starch.

2. The composition according to claim 1, wherein the (A) UV wavelength conversion substance comprises an inorganic UV wavelength conversion substance.

3. The composition according to claim 2, wherein the inorganic UV wavelength conversion substance comprises a magnesium titanate phosphor and/or a zinc oxide phosphor.

4. The composition according to claim 1, wherein the (A) UV wavelength conversion substance comprises an organic UV wavelength conversion substance.

5. The composition according to claim 4, wherein the organic UV wavelength conversion substance comprises one or more selected from the group of phycocyanin, phycoerythrocyanin, phycoerythrin, vitamin A, β-carotene, vitamin K, vitamin B1,vitamin B2, vitamin B2 derivatives, vitamin B6, vitamin B12, folic acid, niacin, salicylic acid, lycopene, gardenia color, capsicum color, capsicum extract, paprika color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a phosphor doped with a sulfur-containing compound, a magnesium titanate phosphor, and a calcium phosphate phosphor.

6. The composition according to any one of claims 1 to 5, comprising both an inorganic UV wavelength conversion substance and an organic UV wavelength conversion substance as the (A) UV wavelength conversion substance.

7. A composition comprising (A') one or more substances selected from the group of phycocyanin, phycoerythrocyanin, phycoerythrin, vitamin A, β-carotene, vitamin K, vitamin B1, vitamin B2, vitamin B2 derivatives, vitamin B6, vitamin B12, folic acid, niacin, salicylic acid, lycopene, gardenia color, capsicum color, capsicum extract, paprika color, safflower color, turmeric color, cochineal color, perilla color, red cabbage color, flavonoid, carotenoid, quinoid, porphyrins, anthocyanins, polyphenols, Red No. 401, Red No. 227, Red No. 504, Red No. 218, Orange No. 205P, Yellow No. 4, Yellow No. 5, Green No. 201, pyranine conc., Blue No. 1, 2,4-diaminophenoxyethanol hydrochloride, Alizuline Purple SS, Purple No. 401, Black No. 401, Herindon pink, Yellow No. 401, Benzidine yellow G, Blue No. 404, Red No. 104, meta-aminophenol; a blue phosphor comprising an amorphous silica particle, cerium, and phosphorus and/or magnesium; a red phosphor comprising a europium-activated compound of a mixed crystal of an alkaline earth metal sulfide and a gallium compound; a zinc oxide phosphor, a phosphor doped with a sulfur-containing compound, a magnesium titanate phosphor, and a calcium phosphate phosphor, and
(B) a hydrophobized silica and/or a hydrophobized starch.

8. The composition according to claim 7, wherein the (A') substance comprises one or more selected from the group of a zinc oxide phosphor, a magnesium titanate phosphor, phycocyanin, and vitamin B2.

9. The composition according to any one of claims 1 to 8, wherein the (B) hydrophobized silica has an average particle size of 1 to 10 µm.

10. The composition according to any one of claims 1 to 9, wherein the (B) hydrophobized silica is fatty acid soap treated silica, fatty acid ester treated silica, higher alcohol treated silica, silicone treated silica, or fatty acid treated silica.

11. The composition according to any one of claims 1 to 10, wherein the (B) hydrophobized starch comprises alkenylsuccinic acid treated starch.

12. The composition according to any one of claims 1 to 11, further comprising (C) a UV absorber and/or (D) a UV scattering agent.

13. The composition according to any one of claims 1 to 12, which is a sunscreen cosmetic.

14. The composition according to any one of claims 1 to 13, which exhibits a fluorescence intensity increasing effect.

15. The composition according to any one of claims 1 to 14, which exhibits a cell activation effect.
